# EUROPEAN PATENT APPLICATION

(11) **EP 4 516 209 A1**
(43) Date of publication of application: **05.03.2025**
(21) Application number: 23862196.5
(22) Date of filing: 24.08.2023
(51) Int. Cl.: A61B 5/00

(54) **PHYSIOLOGICAL DETECTION METHOD BASED ON WEARABLE DEVICE, AND WEARABLE DEVICE**

(30) Priority: 09.09.2022 CN 202211103402
(71) Applicant: Honor Device Co., Ltd., Shenzhen, Guangdong 518040 (CN)
(72) Inventor: ZHANG, Xiaowu, Shenzhen, Guangdong 518040 (CN); LI, Danhong, Shenzhen, Guangdong 518040 (CN); DI, Haoxuan, Shenzhen, Guangdong 518040 (CN)
(74) Representative: MERH-IP Matias Erny Reichl Hoffmann Patentanwälte PartG mbB
(86) International application number: PCT/CN2023/114806
(87) International publication number: WO 2024/051500

(57) **Abstract**

This application provides a wearable device-based physiological detection method and a wearable device, and relates to the field of wearable device technologies. The wearable device includes a multi-source sensor, and the multi-source sensor includes a PPG sensor and at least one motion sensor. After storing M pieces of PPG data in a ring buffer of the PPG sensor, the wearable device may first determine, based on timestamps corresponding to the M pieces of PPG data, a data collection time period corresponding to the M pieces of PPG data. Then, the wearable device separately reads motion data in the data collection time period from a ring buffer of each motion sensor, and if a quantity of pieces of the motion data is greater than a specific numerical value, determines that the motion data is aligned with the M pieces of PPG data, so as to determine multi-source data in a same time period. Then, the wearable device may perform physiological parameter calculation by using the aligned multi-source data, so as to improve accuracy of physiological parameter calculation and avoid impact of motion noise.

## Description

This application claims priority to Chinese Patent Application No. 202211103402.5, filed with the China National Intellectual Property Administration on September 09, 2022 and entitled "WEARABLE DEVICE-BASED PHYSIOLOGICAL DETECTION METHOD AND WEARABLE DEVICE", which is incorporated herein by reference in its entirety.

### TECHNICAL FIELD

This application relates to the field of wearable device technologies, and in particular, to a wearable device-based physiological detection method and a wearable device.

### BACKGROUND

A wearable device such as a smartwatch may detect a physiological parameter (such as a heart rate or blood oxygen) of a wearer. Currently, photoplethysmographic (photoplethysmographic, PPG) is usually used to detect the physiological parameter of the wearer. Based on an LED light source and a detector, the PPG measures attenuated light that is reflected and absorbed by a blood vessel and a tissue of a human body, records a pulsation state of the blood vessel, and measures a pulse wave, to obtain a corresponding physiological parameter. However, for some reasons (for example, when a user is in a motion state during detection), accuracy of a physiological parameter determined only by using PPG data that is collected by a PPG sensor is relatively low. Therefore, the wearable device may determine the physiological parameter of the wearer by using the PPG data collected by the PPG sensor as a core signal, in combination with motion data collected by a motion sensor (for example, an acceleration sensor), that is, by fusing data collected by a multi-source sensor.

However, there may be a time deviation between data collected by different sensors. Therefore, to ensure accuracy of physiological parameter detection, before performing data fusion, the wearable device needs to align the data collected by the multi-source sensor. Then, the wearable device fuses the aligned multi-source data, so as to determine the physiological parameter of the wearer by using the fused multi-source data.

### SUMMARY

In view of this, this application provides a wearable device-based physiological detection method and a wearable device, so as to implement alignment of multi-source data.

According to a first aspect, this application provides a wearable device-based physiological detection method, where the wearable device includes a multi-source sensor, and the multi-source sensor includes a PPG sensor and one or more motion sensors. The electronic device or the wearable device receives a first operation, where the first operation is used to trigger the wearable device to detect a physiological parameter of a wearer of the wearable device, and the physiological parameter includes at least one of the following: a heart rate, blood oxygen, a pulse, and a cardiovascular.

In response to the first operation, the wearable device collects PPG data by using the PPG sensor, and stores, in a first buffer, the PPG data collected by the PPG sensor. In addition, the wearable device collects motion data by using the motion sensor, and separately stores, in one or more second buffers, motion data collected by each motion sensor, where each piece of PPG data collected by the PPG sensor and each piece of motion data collected by the motion sensor separately correspond to one timestamp, which is used to record a collection time of corresponding data.

After collecting M pieces of PPG data in the first buffer, the wearable device separately obtains, from each second buffer based on timestamps corresponding to the M pieces of PPG data, a plurality of pieces of motion data that meet a preset condition, where M is a preset value. The plurality of pieces of motion data that meet the preset condition in each second buffer are continuously collected by a corresponding motion sensor, times recorded by timestamps corresponding to the plurality of pieces of motion data are later than a time recorded by a first timestamp, a time recorded by a timestamp corresponding to at least one piece of motion data is earlier than a time recorded by a second timestamp, and a difference between a quantity of the plurality of pieces of motion data and M is less than a first preset value. The first timestamp is a timestamp with an earliest recording time in the timestamps corresponding to the M pieces of PPG data, and the second timestamp is a timestamp with a latest recording time in the timestamps corresponding to the M pieces of PPG data.

The wearable device obtains and outputs a physiological detection result based on the M pieces of PPG data and the plurality of pieces of motion data that meet the preset condition and that are obtained from the one or more second buffers.

In this application, after the wearable device collects the M pieces of PPG data in the first buffer, it indicates that there is sufficient PPG data to meet a data amount required for physiological parameter calculation. In this case, the wearable device separately obtains, from each second buffer based on the timestamps corresponding to the M pieces of PPG data, the plurality of pieces of motion data that meet the preset condition, so as to obtain motion data in a data collection time period corresponding to the M pieces of PPG data, and determine multi-source data in a same time period, thereby implementing alignment of multi-source data. Then, the wearable device performs physiological detection based on the aligned multi-source data (that is, the M pieces of PPG data and the plurality of pieces of motion data that meet the preset condition in each second buffer), that is, calculates a physiological parameter of a wearer of the wearable device, and outputs a corresponding physiological detection result. Because the physiological parameter is obtained through calculation based on the aligned multi-source data, accuracy of calculating the physiological parameter can be ensured, and accurate detection of the physiological parameter of the wearer can be implemented.

In a possible design, the first buffer and the second buffer are ring buffers. The ring buffer is a data structure of a fixed-size, head-to-tail connected buffer. After one data element in the ring buffer is used and removed, storage locations of remaining data elements do not need to be moved, thereby reducing memory allocation. Therefore, in this application, data is stored by using a ring buffer, so that allocated memory can be fully utilized, and use of memory allocation can be reduced, thereby reducing generation of memory fragments.

In a possible design, a process of obtaining the plurality of pieces of motion data that meet the preset condition from each second buffer is as follows: After collecting the M pieces of PPG data in the first buffer, for each second buffer, the wearable device reads, from the second buffer, motion data in a time interval corresponding to the first timestamp and the second timestamp. The wearable device calculates a difference between M and a quantity of pieces of the motion data in the time interval corresponding to the first timestamp and the second timestamp. If the difference is less than a first preset value corresponding to the second buffer, the wearable device determines that the motion data in the time interval corresponding to the first timestamp and the second timestamp in the second buffer meets a preset condition, so as to determine motion data of the motion sensor in the same time period, and in addition, a data amount of the motion data of the motion sensor in the same time period can also meet a requirement for physiological parameter calculation, thereby implementing alignment between the motion data of the motion sensor and the PPG data of the PPG sensor, that is, implementing alignment processing of multi-source data.

In another possible design, a process of obtaining the plurality of pieces of motion data that meet the preset condition from each second buffer is as follows: After collecting the M pieces of PPG data in the first buffer, for each second buffer, the wearable device reads, from the second buffer, motion data in a time interval corresponding to the first timestamp and the second timestamp. The wearable device determines a timestamp with an earliest recording time in the timestamps corresponding to the motion data in the time interval corresponding to the first timestamp and the second timestamp, so as to obtain a third timestamp of the second buffer. In addition, the wearable device determines a timestamp with a latest recording time in the timestamps corresponding to the motion data in the time interval corresponding to the first timestamp and the second timestamp, so as to obtain a fourth timestamp of the second buffer.

The wearable device calculates a difference between M and a quantity of pieces of the motion data in the time interval corresponding to the first timestamp and the second timestamp. In addition, the wearable device calculates a first time difference between the third timestamp of the second buffer and the first timestamp, and calculates a second time difference between the fourth timestamp of the second buffer and the second timestamp.

If the difference is less than a first preset value corresponding to the second buffer, the first time difference is less than a first preset time difference, and the second time difference is less than a second preset time difference, the wearable device determines that the motion data in the time interval corresponding to the first timestamp and the second timestamp in the second buffer meets the preset condition, thereby implementing accurate alignment of the collection time, and implementing accurate determining of the motion data of the motion sensor in the same time period, and in addition, a data amount of the motion data of the motion sensor in the same time period can also meet a requirement for physiological parameter calculation, thereby implementing accurate alignment between the motion data of the motion sensor and the PPG data of the PPG sensor, that is, implementing alignment processing of multi-source data.

In some implementations, if the foregoing difference between M and the quantity of pieces of the motion data in the time interval corresponding to the first timestamp and the second timestamp in the second buffer is greater than or equal to the first preset value corresponding to the second buffer, it indicates that an amount of the motion data of the motion sensor corresponding to the second buffer in the same time period is relatively small and does not meet an amount requirement for physiological parameter calculation. In this case, the wearable device sends first prompt information, where the first prompt information is used to indicate that the wearable device is collecting physiological data.

In some implementations, if the foregoing difference between M and the quantity of pieces of the motion data in the time interval corresponding to the first timestamp and the second timestamp in the second buffer is greater than or equal to the first preset value corresponding to the second buffer, the wearable device may clear all motion data in the second buffer.

In some implementations, if the first time difference of the second buffer is greater than or equal to a first preset time difference, it indicates that a difference between the first timestamp and the third timestamp of the second buffer is relatively large, and the motion sensor corresponding to the second buffer collects motion data only after a relatively long time elapses after the first timestamp. In this case, the wearable device may clear all motion data in the second buffer.

In some implementations, if the second time difference of the second buffer is greater than or equal to a second preset time difference, it indicates that the motion sensor corresponding to the second buffer may not report the collected motion data in a timely manner. In this case, the wearable device continues to wait, that is, after preset duration elapses, continues to obtain motion from the second buffer for alignment processing.

In a possible design, a frequency of collecting PPG data by the PPG sensor is a first component frequency, and a frequency of collecting motion data by the one or more motion sensors is one or more second component frequencies.

The first component frequency is the same as the one or more second component frequencies, or the first component frequency is different from the one or more second component frequencies. The first preset value depends on a difference between the first component frequency and the one or more second component frequencies. A smaller difference between the first component frequency and the one or more second component frequencies leads to a smaller first preset value. Second component frequencies of collecting motion data by different motion sensors are the same or different.

In a possible design, before the storing, in a first buffer, the PPG data collected by the PPG sensor, the wearable device uses every P pieces of PPG data collected by the PPG sensor as one first data packet, and stores the first data packet and a timestamp of the first data packet in a third buffer. The timestamp of the first data packet is a timestamp corresponding to one piece of PPG data in the P pieces of PPG data, P is a first preset numerical value, and the timestamp of the first data packet is used as a timestamp corresponding to each piece of PPG data in the corresponding P pieces of PPG data.

The wearable device calculates, based on P and times recorded by timestamps of adjacent first data packets in the third buffer, a first actual frequency of collecting PPG data by the PPG sensor.

The storing collected PPG data in a first buffer includes:
if a difference between the first actual frequency and the first component frequency is less than a second preset value, the wearable device buffers the first data packet in the third buffer to the first buffer.

The collecting the M pieces of PPG data in the first buffer includes: collecting K first data packets in the first buffer, where M=K*P.

In this application, to reduce power consumption, the PPG sensor may report the collected PPG data in a form of a data packet, that is, report P pieces of PPG data as one first data packet when collecting the P pieces of PPG data. In a process of reporting a data packet by the PPG sensor, a packet loss phenomenon may occur. Therefore, before alignment processing, the wearable device may first perform frequency offset check on the PPG sensor to determine whether a first data packet is lost. To be specific, the wearable device calculates the first actual frequency of the PPG sensor based on the timestamps of the adjacent first data packets in the third buffer and the quantity P of pieces of PPG data included in the first data packet. If the difference between the first actual frequency of the PPG sensor and the first component frequency of the PPG sensor is less than the second preset value, it indicates that no packet loss phenomenon occurs. In this case, the wearable device may buffer the first data packet in the third buffer to the first buffer.

In some implementations, if the foregoing difference between the first actual frequency and the first component frequency is less than the second preset value, it indicates that a packet loss phenomenon occurs. In this case, the wearable device sends second prompt information, where the second prompt information is used to indicate that physiological data of the wearable device is abnormal.

In a possible design, the foregoing one or more motion sensors include an acceleration sensor and a gyroscope. The one or more second buffers include an acceleration buffer configured to store first motion data collected by the acceleration sensor and a gyroscope buffer configured to store second motion data collected by the gyroscope. Correspondingly, the wearable device may also perform frequency offset check on the acceleration sensor and the gyroscope.

For example, a process of performing frequency offset check on the acceleration sensor is as follows:

Before the separately storing, in one or more second buffers, motion data collected by each motion sensor, the wearable device uses every Q pieces of first motion data collected by the acceleration sensor as one second data packet, and stores the second data packet and a timestamp of the second data packet in a fourth buffer. The timestamp of the second data packet is a timestamp corresponding to one piece of first motion data in the Q pieces of first motion data, Q is a second preset numerical value, and the timestamp of the second data packet is used as a timestamp corresponding to each piece of motion data in the corresponding Q pieces of first motion data.

The wearable device calculates, based on Q and times recorded by timestamps of adjacent second data packets in the fourth buffer, a second actual frequency of collecting the first motion data by the acceleration sensor.

Storing the first motion data in the acceleration buffer includes:
if a difference between the second actual frequency and the second component frequency corresponding to the acceleration sensor is less than a third preset value, the wearable device buffers the second data packet in the fourth buffer to the second buffer.

The plurality of pieces of first motion data that meet the preset condition include W second data packets, where N=W*Q, and N is a quantity of pieces of the first motion data in the plurality of pieces of first motion data that meet the preset condition.

In this application, after obtaining each second data packet reported by the acceleration sensor, the wearable device calculates, based on the second data packet and a previous second data packet of the second data packet, that is, based on timestamps of two adjacent second data packets and the quantity Q of pieces of first motion data included in the second data packet, an actual frequency of collecting the second data packet by the acceleration sensor, that is, obtains the foregoing second actual frequency. The wearable device calculates a difference between the second actual frequency and the second component frequency corresponding to the acceleration sensor. If the difference is less than a third preset value, it indicates that the second actual frequency of the acceleration sensor is consistent with a standard frequency of the acceleration sensor, that is, it indicates that no second data packet is lost between the two adjacent second data packets, that is, it indicates that no packet loss occurs on the acceleration sensor, and the second data packet may be used for alignment. In this case, the wearable device may buffer the second data packet to the second buffer corresponding to the acceleration sensor, so that the second buffer stores motion data that can be used for alignment processing, thereby avoiding accumulation of frequency offset errors, avoiding interference of abnormal motion data, and ensuring alignment accuracy.

In a possible design, after determining, based on the timestamp corresponding to the M pieces of PPG data, the plurality of pieces of motion data that meet the preset condition, the wearable device stores the M pieces of PPG data and the plurality of pieces of motion data that meet the preset condition in a fifth buffer. In other words, the fifth buffer stores the aligned multi-source data, so that the wearable device may directly read the aligned multi-source data from the fifth buffer, calculate a physiological parameter of the wearer of the wearable device based on the aligned multi-source data, and output a corresponding physiological detection result based on the physiological parameter, so that the aligned multi-source data is quickly read, thereby improving calculation efficiency of the physiological parameter and improving generation efficiency of the physiological detection result.

According to a second aspect, this application provides a wearable device, where the wearable device includes a display screen, a memory, a multi-source sensor, and one or more processors. The display screen, the multi-source sensor, and the memory are coupled to the processor. The display screen is configured to display an image generated by the processor, the multi-source sensor includes a PPG sensor and one or more motion sensors, the memory is configured to store computer program code, and the computer program code includes computer instructions. When the processor executes the computer instructions, the wearable device is enabled to perform the foregoing method.

According to a third aspect, this application provides a computer storage medium, including computer instructions. When the computer instructions are run on a wearable device, the wearable device is enabled to perform the foregoing method.

According to a fourth aspect, this application provides a computer program product. When the computer program product runs on a wearable device, the wearable device is enabled to perform the foregoing method.

It may be understood that, for beneficial effects that can be achieved by the wearable device according to the second aspect, the computer storage medium according to the third aspect, and the computer program product according to the fourth aspect that are provided above, reference may be made to the beneficial effects in the first aspect and any possible design manner of the first aspect. Details are not described herein again.

### BRIEF DESCRIPTION OF DRAWINGS

FIG. 1 is a schematic diagram of displaying a physiological parameter according to an embodiment of this application;
FIG. 2 is a schematic diagram of a hardware structure of a wearable device according to an embodiment of this application;
FIG. 3 is a schematic diagram of a software architecture of a wearable device according to an embodiment of this application;
FIG. 4 is a schematic flowchart of a multi-source alignment method according to an embodiment of this application;
FIG. 5 is a schematic diagram 1 of triggering monitoring according to an embodiment of this application;
FIG. 6 is a schematic diagram 2 of triggering monitoring according to an embodiment of this application;
FIG. 7 is a schematic diagram of displaying first prompt information according to an embodiment of this application;
FIG. 8 is a schematic diagram of displaying second prompt information according to an embodiment of this application;
FIG. 9 is a schematic diagram of a process of calculating a physiological detection result according to an embodiment of this application; and
FIG. 10 is a schematic diagram of processes of preprocessing and alignment processing according to an embodiment of this application.

### DESCRIPTION OF EMBODIMENTS

In the following, the terms "first" and "second" are used merely for the purpose of description, and should not be construed as indicating or implying relative importance or implicitly indicating a quantity of indicated technical features. Therefore, a feature defined as "first" or "second" may explicitly or implicitly include one or more of the features. In the descriptions of the embodiments, unless otherwise stated, "a plurality of" means two or more.

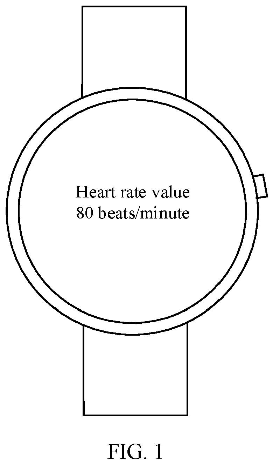

A wearable device such as a smartwatch may detect a physiological parameter of a wearer, and display the physiological parameter (as shown in FIG. 1, the wearable device displays a heart rate of the wearer), so that the wearer learns of a current physiological condition.

The wearable device may obtain, through calculation, the physiological parameter of the wearer by using a PPG signal (that is, PPG data) collected by a PPG sensor. However, during PPG signal collection, the wearer may be in a motion state, that is, the wearable device may be in a non-still state. Noise of motion seriously interferes with quality of the PPG signal, and causes problems such as a motion artifact and a PPG waveform change. Therefore, physiological parameters such as a heart rate, blood oxygen, a pulse, and a cardiovascular cannot be accurately calculated only by using the PPG signal. To improve accuracy of determining a physiological parameter, the wearable device may first fuse data collected by a multi-source sensor in the wearable device, that is, fuse PPG data collected by the PPG sensor and motion data collected by the motion sensor. Then, the wearable device may denoise (that is, remove motion noise from) the fused data, and extract a real PPG waveform, so that a physiological parameter can be calculated by using the real PPG waveform, thereby ensuring accuracy of the physiological parameter.

For example, the foregoing motion sensor may include a gyroscope (gyroscope, gyro) sensor and/or an acceleration (acceleration, acc) sensor.

However, because the multi-source sensors are different components, and component frequencies of the sensors in the multi-source sensors are different, that is, data is not reported at a same time. In other words, timestamps at which the sensors report the data collected by the sensors may be inconsistent, and consequently, a time deviation between data of different sensors received by the wearable device is relatively large. For example, a timestamp of PPG data received by the wearable device is 10:20:30 AM, and a timestamp of motion data is 10:21:40 AM. The times of the PPG data and the motion data differ greatly, and are not data collected in a same time period. Consequently, accuracy of a physiological parameter obtained through calculation based on the fused data of the PPG data and the motion data is relatively low. In addition, amounts of data received from different sensors may differ greatly. For example, when collecting 100 pieces of PPG data, the wearable device collects 60 pieces of motion data. The amounts of the PPG data and the motion data differ greatly, and cannot be well fused and denoised. Consequently, accuracy of extracting a PPG waveform based on the fused and denoised data cannot be ensured, that is, accuracy of calculating a physiological parameter cannot be ensured.

Therefore, to resolve the foregoing problem, this application provides a wearable device-based multi-source data alignment method. The wearable device includes a multi-source sensor, a first buffer, and a second buffer. The multi-source sensor includes a PPG sensor and a motion sensor, and the first buffer is configured to store PPG data collected by the PPG sensor. A quantity of the second buffers is in a one-to-one correspondence with a type of a motion sensor. Each second buffer is configured to store motion data collected by a corresponding motion sensor. When the PPG data in the first buffer reaches a specific amount, it indicates that the PPG data is sufficient, and may be used to calculate a physiological parameter. In this case, the wearable device may first determine a data collection time period based on a timestamp of the PPG data in the first buffer. Then, the wearable device obtains motion data whose timestamp is in the data collection time period from each second buffer, so as to obtain the motion data in the same time period. In addition, if the motion data in the data collection time period meets a requirement, it indicates that an amount of the motion data differs slightly from the amount of the PPG data, and the motion data may be used to calculate a physiological parameter, so as to implement alignment processing of multi-source data (that is, the motion data and the PPG data). Then, the wearable device may perform fusion and denoising by using the aligned multi-source data, extract a real PPG waveform, and calculate a corresponding physiological parameter by using the real PPG waveform, thereby ensuring accuracy of physiological parameter calculation. In addition, data is stored by using a buffer, so that allocated memory can be fully utilized, and use of memory allocation can be reduced, thereby reducing generation of memory fragments.

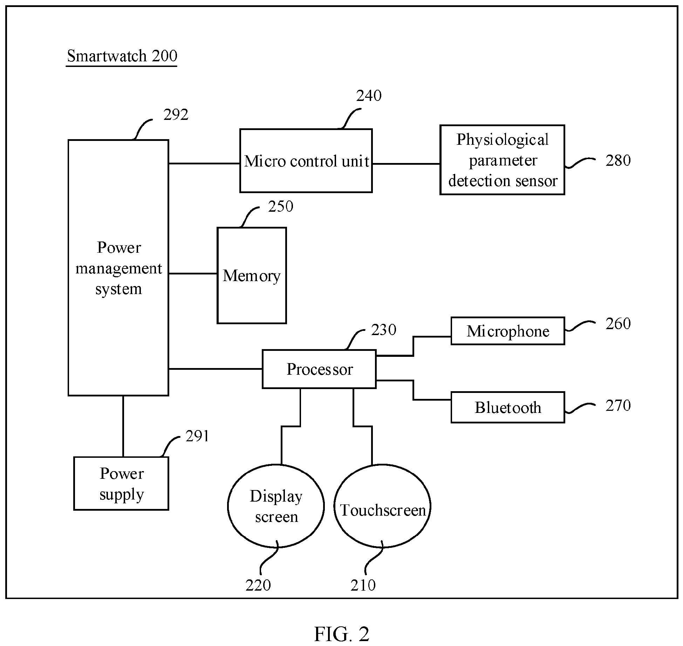

For example, the wearable device is a device that can collect a physiological parameter of the wearer and that is in contact with the wearer, such as a smartwatch or a smart band. With reference to FIG. 2, the following describes a hardware structure of the wearable device by using an example in which the wearable device is a smartwatch.

As shown in FIG. 2, the smartwatch 200 includes a watch body and a wrist strap that are connected to each other. The watch body may include a front housing (not shown in FIG. 2), a touchscreen 210 (also referred to as a touch panel), a display screen 220, a bottom housing (not shown in FIG. 2), a processor 230, a micro control unit (micro control unit, MCU) 240, a memory 250, a microphone (Microphone, MIC) 260, a Bluetooth (bluetooth, BT) module 270, a physiological parameter detection sensor 280, a power supply 291, a power management system 292, and the like. Although not shown, the smartwatch may further include an antenna, a WiFi module, a GPS module, a loudspeaker, an accelerometer, a gyroscope, an acceleration sensor, and the like. A person skilled in the art may understand that the structure of the smartwatch shown in FIG. 2 does not constitute a limitation on the smartwatch. The smartwatch may include more or fewer components than those shown in the figure, or combine some components, or have different component arrangements.

The following separately describes functional components of the smartwatch 200.

The touch panel 210, also referred to as a touchpad, may collect a touch operation of a user of the watch on the touch panel (such as an operation of the user on or near the touch panel by using any suitable object or accessory such as a finger or a stylus), and drive a responding connection apparatus based on a preset program. Optionally, the touch panel 210 may include two parts: a touch detection apparatus and a touch controller. The touch detection apparatus detects a touch direction of the user and a signal brought by the touch operation, and sends the signal to the touch controller. The touch controller receives touch information from the touch detection apparatus, converts the touch information into touch point coordinates, sends the touch point coordinates to the processor 230, and can receive and execute a command sent by the processor 230. In addition, the touch panel may be implemented as a resistive type, a capacitive type, an infrared type, a surface acoustic wave type, or the like. In addition to the touch panel 210, the smartwatch may further include another input device. The another input device may include but is not limited to a function key (such as a volume control key or an on/off key).

The display screen 220 may be configured to display information inputted by the user or information provided for the user, and various menus of the watch. Optionally, the display screen 220 may be configured in a form of an LCD, an OLED, or the like. Further, the touch panel 210 may cover the display screen 220. After detecting a touch operation on or near the touch panel 210, the touch panel transfers the touch operation to the processor 230, to determine a type of a touch event. Then, the processor 230 provides a corresponding visual output on the display screen 220 based on the type of the touch event. Although in FIG. 2, the touch panel 210 and the display screen 220 respectively implement the input and output functions of the watch as two independent components, in some embodiments, the touch panel 210 and the display screen 220 may be integrated to implement the input and output functions of the watch.

The processor 230 is configured to: perform system scheduling, control the display screen and the touchscreen, and support processing of the microphone 360, one or more thin film actuators, the Bluetooth module 270, and the like.

Bluetooth module 270: The smartwatch may exchange information with another electronic device (such as a mobile phone or a tablet computer) by using the Bluetooth module 270.

The micro control unit 240 is configured to: control a sensor, perform an operation on sensor data, communicate with the processor 230, and perform another function.

The sensor may be a physiological parameter detection sensor 280, a barometric pressure sensor, a gravity sensor, a light sensor, a motion sensor, or another sensor. Specifically, the light sensor may include an ambient light sensor and a proximity sensor. For other sensors such as an acceleration sensor, a gyroscope, a barometer, a hygrometer, a thermometer, and an infrared sensor that may be further configured on the watch, details are not described herein again.

The physiological parameter detection sensor 280 may include a PPG sensor and the like.

The memory 250 may be configured to store a software program and data. The processor 230 performs various function applications and data processing of the watch by running the software program and the data that are stored in the memory. The memory 250 mainly includes a program storage area and a data storage area. The program storage area may store an operating system and an application program required by at least one function (for example, a sound playing function and an image playing function). The data storage area may store data (for example, audio data and a phone book) created based on the use of the watch. In addition, the memory may include a high-speed random access memory, and may further include a non-volatile memory, for example, a magnetic disk storage device, a flash memory device, or another volatile solid-state storage device.

The smartwatch 200 further includes the power supply 291 (such as a battery) for supplying power to the components. Optionally, the power supply 291 may be logically connected to the processor 230 by using the power management system 292, thereby implementing functions such as charging, discharging, and power consumption management by using the power management system 292.

It should be understood that the smartwatch 200 shown in the figure is only an example of the wearable device, and the smartwatch 200 may have more or fewer components than those shown in the figure, or may combine two or more components, or may have different component configurations. The components shown in FIG. 2 may be implemented by hardware that includes one or more signal processing and/or application-specific integrated circuits, software, or a combination of hardware and software.

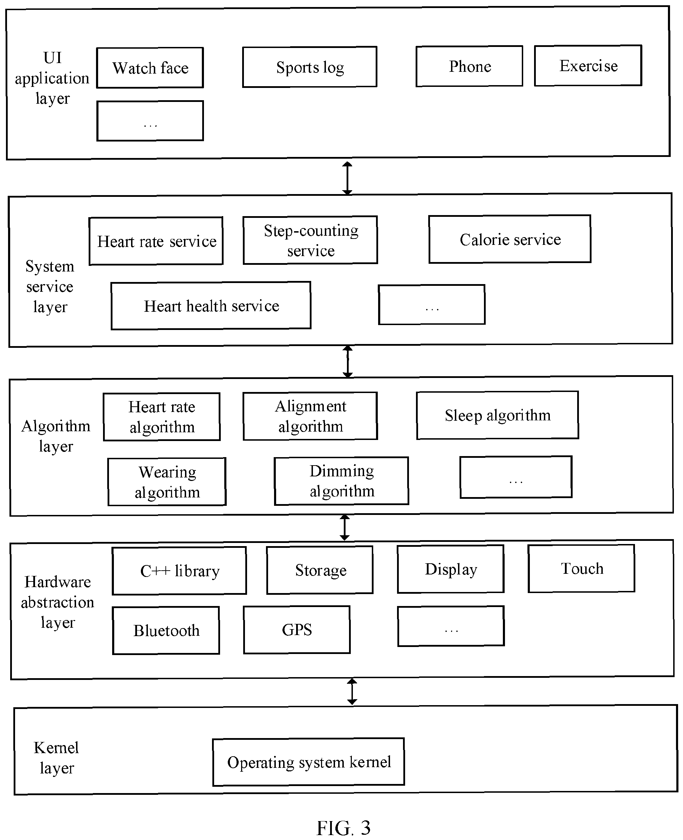

FIG. 3 is a block diagram of a software structure of a wearable device according to an embodiment of this application. In a layered architecture of the wearable device, software is divided into several layers, and each layer has a clear role and task. The layers communicate with each other through software interfaces. For example, in some embodiments, the wearable device is a smartwatch. As shown in FIG. 3, an operating system of the smartwatch may include five layers from top to bottom: a UI (User Interface, user interface) application layer, a system service layer, an algorithm layer, a hardware abstraction layer, and a kernel layer.

The UI application layer may include a series of application packages, for example, Watch face, Sports log, Phone, and Exercise.

The system service layer may include a series of system services. As shown in FIG. 3, the system service layer may include a heart rate service. The heart rate service may provide physiological parameter information of a smartwatch wearer, for example, a heart rate, a blood pressure, blood oxygen, a pulse rate (pulse for short), a respiration rate, and a temperature, and may further detect physiological parameter change information of the smartwatch wearer. The system service layer may further include a step-counting service, a calorie service, and a heart health service.

The algorithm layer may include a series of algorithm models. As shown in FIG. 3, the algorithm layer may include a heart rate algorithm model and a multi-source data alignment algorithm model. The heart rate algorithm model is used to calculate a physiological parameter of the smartwatch wearer, and the multi-source data alignment algorithm model is used to align data collected by the multi-source sensor. The algorithm layer may further include a sleep algorithm model, a wearing algorithm model, a dimming algorithm model, and the like.

The hardware abstraction layer (hardware abstraction layer, HAL) is an interface layer located between an operating system kernel and a hardware circuit. As shown in FIG. 3, the HAL layer includes a storage HAL, a display HAL, a touch HAL, a Bluetooth HAL, and a global positioning system (global positioning system, GPS) HAL. The storage HAL may be used to perform storage processing on the data collected by the sensor. The display HAL may be used to display information. The touch HAL may be the same as detecting a user-triggered operation. The Bluetooth HAL may be used to implement a Bluetooth communication function. The GPS HAL may be used to implement a positioning function. This HAL layer may be implemented by using a C++ library.

As shown in FIG. 3, the kernel layer includes at least an operating system kernel.

It may be understood that, the layers and components included in the layers in the software structure shown in FIG. 3 do not constitute a specific limitation on the smartwatch. In some other embodiments of this application, the smartwatch may include more or fewer layers than those shown in the figure, and each layer may include more or fewer components. This is not limited in this application.

This application provides a wearable device-based multi-source data alignment method. When determining the physiological parameter of the wearer of the wearable device in a multi-source data fusion manner, the wearable device uses PPG data collected by the PPG sensor as core data to fuse motion data collected by the motion sensor. However, because different sensors report data at different frequencies, to ensure accuracy of physiological parameter calculation, the wearable device needs to align the multi-source data before fusing the multi-source data, so as to obtain a plurality of pieces of multi-source data in a same time period. Then, the wearable device fuses and denoises the plurality of pieces of multi-source data, and extracts a real PPG waveform, so as to calculate a corresponding physiological parameter by using the real PPG waveform, and avoid relatively low accuracy of physiological parameter calculation due to motion noise.

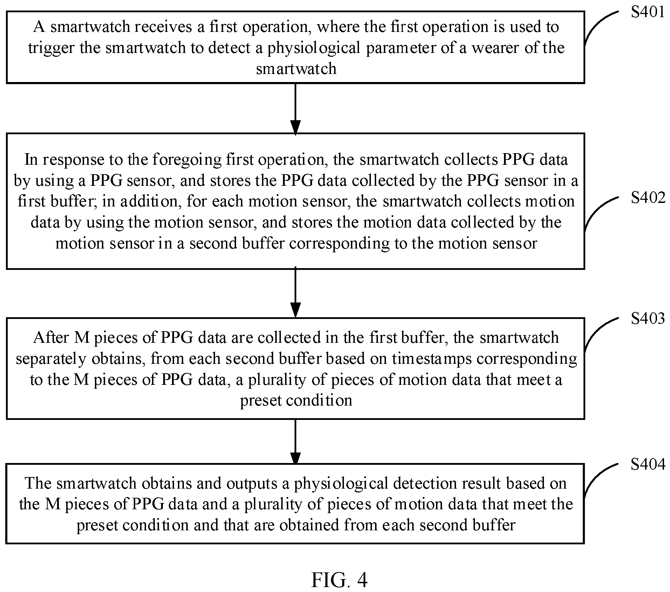

With reference to the accompanying drawings, the following describes in detail the multi-source data alignment method provided in the embodiments of this application by using an example in which the wearable device is a smartwatch. As shown in FIG. 4, the method includes the following steps:

S401. The smartwatch receives a first operation, where the first operation is used to trigger the smartwatch to detect a physiological parameter of a wearer of the smartwatch.

The foregoing physiological parameter includes one or more of a heart rate, blood oxygen, a pulse, and a cardiovascular. For example, the cardiovascular physiological parameter may be a blood pressure, and the blood pressure is a side pressure of blood in a blood vessel against a blood vessel wall. The physiological parameter of blood oxygen may be blood oxygen saturation.

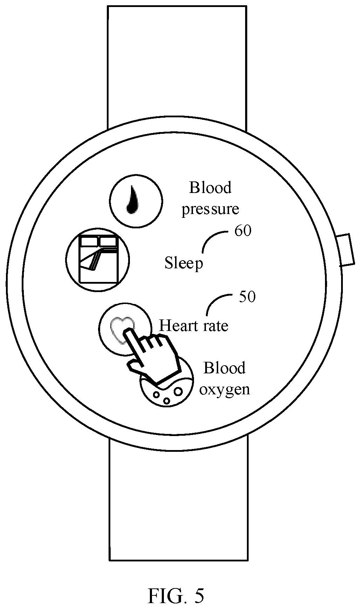

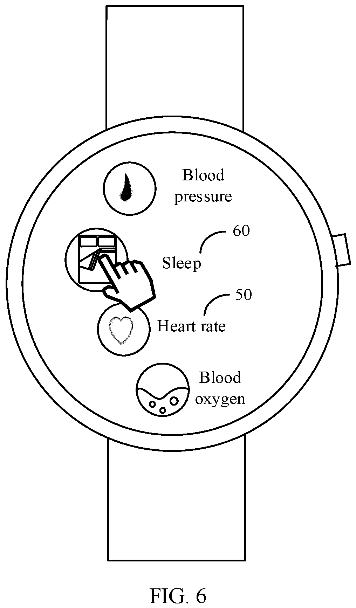

For example, when a user wants the smartwatch to perform specific health monitoring, the health monitoring needs to detect a physiological parameter of the wearer, and the user may tap a related health function control in an interface displayed on the smartwatch. Herein, tapping the related health function control in the interface by the user is the foregoing first operation. For example, as shown in FIG. 5 and FIG. 6, the interface displayed on the smartwatch includes a heart rate monitoring control 50 and a sleep monitoring control 60. When the user wants the smartwatch to monitor a heart rate of the wearer, the user may tap the heart rate monitoring control 50 (as shown in FIG. 5) in the interface displayed on the smartwatch. Herein, tapping the heart rate monitoring control by the user is the first operation. The first operation is used to trigger the smartwatch to detect the heart rate of the wearer. In other words, the first operation is used to trigger the smartwatch to implement the heart rate monitoring function. For another example, when the user wants the smartwatch to monitor sleep quality of the wearer, the user may tap the sleep monitoring control 60 (as shown in FIG. 6) in the interface displayed on the smartwatch. Herein, an operation of tapping the sleep monitoring control by the user is the first operation. The first operation is used to trigger the smartwatch to detect a physiological parameter of the wearer, so as to determine the sleep quality of the wearer by using the physiological parameter of the wearer. In other words, the first operation is used to trigger the smartwatch to implement the sleep quality monitoring function.

It should be understood that the first operation listed above is only an example. The first operation may alternatively be another type of operation. For example, the user triggers specific hardware in the smartwatch to trigger the smartwatch to detect a physiological parameter of the wearer of the smartwatch. In this application, a type of the first operation is not limited, provided that the first operation can trigger the smartwatch to detect the physiological parameter of the wearer of the smartwatch.

S402. In response to the foregoing first operation, the smartwatch collects PPG data by using a PPG sensor, and stores the PPG data collected by the PPG sensor in a first buffer. In addition, for each motion sensor, the smartwatch collects motion data by using the motion sensor, and stores the motion data collected by the motion sensor in a second buffer corresponding to the motion sensor.

Each piece of PPG data collected by the PPG sensor and each piece of motion data collected by the motion sensor separately correspond to one timestamp, which is used to record a collection time of corresponding data.

Generally, the foregoing motion sensor includes an acceleration sensor and/or a gyroscope. The acceleration sensor is configured to collect an acceleration of the smartwatch, and the gyroscope is configured to collect an angular velocity of the smartwatch. Correspondingly, the motion data collected by the motion sensor includes acceleration data and/or angular velocity data of the smartwatch. Certainly, if another type of motion data is further used when detecting a physiological parameter of the wearer of the smartwatch, the motion sensor may further include a corresponding type of motion sensor. The type of the sensor included in the foregoing motion sensor is not limited in this application.

Both the motion sensor and the PPG sensor in the foregoing smartwatch collect and report data at a specific frequency. The smartwatch may allocate a buffer (that is, a first buffer) to the PPG sensor in the smartwatch, and the first buffer is configured to store PPG data collected by the PPG sensor. In addition, the smartwatch may allocate a corresponding buffer (that is, a second buffer) to each motion sensor in the smartwatch, and the second buffer is configured to store motion data collected by the corresponding motion sensor. For example, a motion sensor used to detect a physiological parameter includes an acceleration sensor and a gyroscope. In this case, the smartwatch allocates a second buffer to the acceleration sensor, and allocates a second buffer to the gyroscope. The second buffer corresponding to the acceleration sensor is configured to store motion data (for example, an acceleration of the smartwatch) collected by the acceleration sensor, and the second buffer corresponding to the gyroscope is configured to store motion data (for example, an angular velocity of the smartwatch) collected by the gyroscope.

The buffer is a first-in-first-out queue. In one case, the first buffer and the second buffer are ring buffers (ring buffer). The ring buffer is a data structure used to represent a fixed-size, head-to-tail connected buffer. Corresponding PPG data or motion data is stored by using the ring buffer, so that allocated memory can be fully utilized, and use of memory allocation is reduced, thereby reducing generation of memory fragments.

In another case, the first buffer and the second buffer may alternatively be linear buffers (buffer), and data in the linear buffers is arranged and read in sequence. To store the corresponding PPG data or motion data by using the linear buffer, the smartwatch may need to consider memory management, and may need to perform memory allocation frequently, increasing a system and increasing memory fragments.

In this embodiment of this application, after obtaining the PPG data collected by the PPG sensor, the smartwatch may store the PPG data collected by the PPG sensor in the first buffer. In addition, for each motion sensor, after obtaining the motion data collected by the motion sensor, the smartwatch stores the motion data in the second buffer corresponding to the motion sensor.

S403. After M pieces of PPG data are collected in the first buffer, the smartwatch separately obtains, from each second buffer based on timestamps corresponding to the M pieces of PPG data, a plurality of pieces of motion data that meet a preset condition.

M is a preset value, and the plurality of pieces of motion data that meet the preset condition in each second buffer are that a timestamp corresponding to each piece of motion data in the plurality of pieces of motion data is later than a first timestamp and earlier than a second timestamp, and a difference between a quantity of the plurality of pieces of motion data and M is less than a first preset value. The first timestamp is a timestamp with an earliest time in the timestamps corresponding to the M pieces of PPG data, and the second timestamp is a timestamp with a latest time in the timestamps corresponding to the M pieces of PPG data. The preset value is a positive integer, and may be set based on an actual requirement. For example, the preset value is 100.

In this embodiment of this application, when the quantity of pieces of PPG data stored in the first buffer is M, it indicates that sufficient PPG data has been stored. The smartwatch may use the M pieces of PPG to perform physiological parameter detection. To improve accuracy of the physiological parameter detection, the smartwatch first obtains a timestamp with an earliest recording time in the timestamps corresponding to the M pieces of PPG data and uses the timestamp as a first timestamp, and obtains a timestamp with a latest recording time in the timestamps corresponding to the M pieces of PPG data and uses the timestamp as a second timestamp. In other words, PPG data corresponding to the first timestamp is PPG data first collected by the PPG sensor in the M pieces of PPG data, and PPG data corresponding to the second timestamp is PPG data last collected by the PPG sensor in the M pieces of PPG data.

Then, the smartwatch uses a time interval corresponding to the first timestamp and the second timestamp as a data collection time period. For example, if the first timestamp is a time point 1 and the second timestamp is a time point 2, the data collection time period is a time interval from the time point 1 to the time point 2.

Then, for each second buffer, the smartwatch obtains, from the second buffer, motion data whose timestamp is in the data collection time period, so as to ensure that the collection time is aligned. In addition, the smartwatch counts a quantity of pieces of motion data in the data collection time period in the second buffer.

Then, the smartwatch calculates a difference between M and the quantity of pieces of motion data in the data collection time period in the second buffer, to obtain a data deviation amount of the motion sensor corresponding to the second buffer. Next, in one case, if the data deviation amount of the motion sensor corresponding to each second buffer is less than a first preset value corresponding to the motion sensor corresponding to each second buffer, it indicates that an amount of motion data that is collected by the motion sensor and that is stored in each second buffer is relatively large, and a data amount of the motion data in the data collection time period in each second buffer is aligned with the data amount of the PPG data, that is, a data amount requirement for physiological parameter detection is met. In this case, the smartwatch may separately use the motion data in the data collection time period in each second buffer as the plurality of pieces of motion data that meet the preset condition in each second buffer, so that a plurality of pieces of motion data in a same time period are determined, thereby implementing alignment of multi-source data.

In another case, for each second buffer, the smartwatch obtains a timestamp with an earliest recording time in the timestamps corresponding to the motion data in the data collection time period in the second buffer, and uses the timestamp as a third timestamp of the second buffer. In other words, the motion data corresponding to the third timestamp of the second buffer is motion data first collected by the motion sensor corresponding to the second buffer in the data collection time period in the second buffer. If a time difference between the first timestamp and the third timestamp of each second buffer is less than a first preset time difference, and the data deviation amount of the motion sensor corresponding to each second buffer is less than a first preset value corresponding to the motion sensor corresponding to each second buffer, it indicates that a collection start time corresponding to the PPG data is highly consistent with a collection start time corresponding to the motion data in the data collection time period in each second buffer, thereby ensuring alignment of the collection time to some extent and meeting a data amount requirement for physiological parameter detection. In this case, the smartwatch may separately use the motion data in the data collection time period in each second buffer as the plurality of pieces of motion data that meet the preset condition in each second buffer.

In another case, for each second buffer, the smartwatch obtains a timestamp with a latest recording time in the timestamps corresponding to the motion data in the data collection time period in the second buffer, and uses the timestamp as a fourth timestamp of the second buffer. In other words, the motion data corresponding to the fourth timestamp of the second buffer is motion data last collected by the motion sensor corresponding to the second buffer in the data collection time period in the second buffer. If a time difference between the second timestamp and the fourth timestamp of each second buffer is less than a second preset time difference, and the data deviation amount of the motion sensor corresponding to each second buffer is less than a first preset value corresponding to the motion sensor corresponding to each second buffer, it indicates that a collection end time corresponding to the PPG data is highly consistent with a collection end time corresponding to the motion data in the data collection time period in each second buffer, thereby ensuring alignment of the collection time to some extent and meeting a data amount requirement for physiological parameter detection. In this case, the smartwatch may separately use the motion data in the data collection time period in each second buffer as the plurality of pieces of motion data that meet the preset condition in each second buffer.

In another case, for each second buffer, the smartwatch obtains a timestamp with an earliest recording time in the timestamps corresponding to the motion data in the data collection time period in the second buffer, and uses the timestamp as a third timestamp of the second buffer. In addition, the smartwatch obtains a timestamp with a latest recording time in the timestamps corresponding to the motion data in the data collection time period in the second buffer, and uses the timestamp as a fourth timestamp of the second buffer. In other words, the motion data corresponding to the third timestamp of the second buffer is motion data first collected by the motion sensor corresponding to the second buffer in the data collection time period in the second buffer, and the motion data corresponding to the fourth timestamp of the second buffer is motion data last collected by the motion sensor corresponding to the second buffer in the data collection time period in the second buffer. If a time difference between the first timestamp and the third timestamp of each second buffer is less than a first preset time difference, a time difference between the second timestamp and the fourth timestamp of each second buffer is less than a second preset time difference, and the data deviation amount of the motion sensor corresponding to each second buffer is less than a first preset value corresponding to the motion sensor corresponding to each second buffer, it indicates that a time interval corresponding to the third timestamp and the fourth timestamp of each second buffer is highly consistent with the data collection time period, that is, the collection time is more aligned, and a data amount requirement for physiological parameter detection is met. In this case, the smartwatch may use the motion data in each second buffer as the plurality of pieces of motion data that meet the preset condition, thereby improving accuracy of multi-source data fusion, and further improving accuracy of physiological parameter detection.

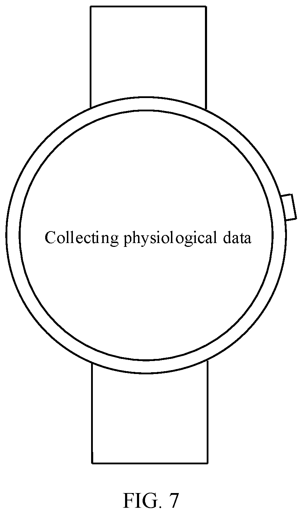

In addition, in the foregoing case, if a data deviation amount of the motion sensor corresponding to a second buffer is greater than or equal to the first preset value, it indicates that a difference between a data amount of PPG data in the first buffer and a data amount of motion data in the data collection time period in the second buffer is relatively large due to some reasons (for example, the motion sensor is abnormal), that is, an amount of the motion data that is collected by the motion sensor and that is stored in the second buffer is relatively small, and the PPG data and the motion data cannot be well fused. In this case, the smartwatch may determine that the motion data in the data collection time period in the second buffer does not meet the preset condition. In addition, the smartwatch may clear the PPG data in the first buffer and all motion data in each second buffer, wait for the PPG sensor and each motion sensor to collect data again, store new PPG data collected by the PPG sensor in the first buffer, and store new motion data collected by each motion sensor in the corresponding second buffer, so that the smartwatch continues to perform alignment processing on multi-source data by using the PPG data in the first buffer and the motion data in each second buffer. Additionally or alternatively, the smartwatch may send first prompt information, where the first prompt information is used to indicate that the wearable device is collecting physiological data (as shown in FIG. 7).

If the time difference between the first timestamp and the third timestamp of the second buffer is greater than or equal to the first preset time difference, it indicates that the difference between the first timestamp and the third timestamp of the second buffer is relatively large. The motion sensor corresponding to the second buffer collects motion data only after a relatively long time elapses after the first timestamp, that is, it indicates that consistency between the collection start time corresponding to the M pieces of PPG data and the collection start time corresponding to the motion data in the data collection time period in the second buffer is relatively low, and collection times of the PPG data and the motion data are not aligned. Therefore, the smartwatch determines that the motion data in the data collection time period in the second buffer does not meet the preset condition, that is, the motion data in the data collection time period in the second buffer cannot be fused with the PPG data to implement accurate physiological parameter detection. In addition, the smartwatch may clear the PPG data in the first buffer and motion data in each second buffer, wait for the PPG sensor and each motion sensor to collect data again, store new PPG data collected by the PPG sensor in the first buffer, and store new motion data collected by each motion sensor in the corresponding second buffer.

If the time difference between the second timestamp and the fourth timestamp of the second buffer is greater than the second preset time difference, it indicates that the difference between the second timestamp and the fourth timestamp of the second buffer is relatively large. The motion sensor corresponding to the second buffer does not collect motion data in a relatively long time before the second timestamp, that is, it indicates that consistency between the collection end time corresponding to the M pieces of PPG data and the collection end time corresponding to the motion data in the data collection time period in the second buffer is relatively low, and collection times of the PPG data and the motion data are not aligned. Therefore, the smartwatch determines that the motion data in the data collection time period in the second buffer does not meet the preset condition, that is, the motion data in the data collection time period in the second buffer cannot be fused with the PPG data to implement accurate physiological parameter detection. In addition, this case may be caused when the motion sensor corresponding to the second buffer does not report the collected motion data in a timely manner. Therefore, the smartwatch may continue to wait for the motion sensor corresponding to the second buffer to report the collected motion data, so as to update the motion data in the data collection time period in the second buffer, so that the smartwatch determines whether the updated motion data in the data collection time period in the second buffer meets the preset condition.

In some embodiments, if the smartwatch clears the data in the first buffer and the second buffer because the motion data in the foregoing data collection time period in the second buffer does not meet the preset condition, and consequently physiological parameter detection cannot be implemented by using the data in the first buffer and the second buffer, the smartwatch cannot display a corresponding physiological parameter, or the physiological parameter displayed by the smartwatch is not updated all the time.

In some embodiments, when determining that the motion data in the foregoing data collection time period in each second buffer meets the preset condition, it indicates that the motion data in the foregoing data collection time period in each second buffer is aligned with the foregoing M pieces of PPG data. In this case, the smartwatch may store the aligned motion data in the foregoing data collection time period in each second buffer and the aligned M pieces of PPG data in a buffer (buffer) at an algorithm layer, and the buffer at the algorithm layer may serve as a fifth buffer, so that the smartwatch may directly read, from the fifth buffer, data that can be used for physiological parameter detection, thereby improving efficiency of obtaining the aligned multi-source data.

For example, the smartwatch may store the aligned motion data in the data collection time period in each second buffer and the aligned M pieces of PPG data in one fifth buffer (buffer), so as to reduce a quantity of buffers that need to be created. Certainly, the smartwatch may alternatively store the aligned motion data in the data collection time period in each second buffer and the aligned M pieces of PPG data in different fifth buffers (buffer). For example, the M pieces of PPG data are stored in one fifth buffer. The fifth buffer is a sensor corresponding to the PPG sensor, and is configured to store the aligned PPG data. Each motion sensor has a corresponding fifth buffer. The fifth buffer corresponding to the motion sensor is configured to store the aligned motion data in the foregoing data collection time period in the second buffer corresponding to the motion sensor.

In some embodiments, after storing the motion data in the foregoing data collection time period in each second buffer and the M pieces of PPG data, the smartwatch may delete the motion data in the data collection time period in the corresponding second buffer and the M pieces of PPG data in the first buffer, thereby reducing resource occupation.

In some embodiments, to reduce system overheads and power consumption of the smartwatch, the data collected by the foregoing sensor is reported in a form of a data packet. Specifically, the PPG data collected by the PPG sensor is reported in a form of a data packet, that is, the smartwatch buffers, in the first buffer, the PPG data in the PPG data packet collected by the PPG sensor. The PPG data packet may include P pieces of PPG data. In other words, the PPG sensor collects the P pieces of PPG data before reporting. The motion data collected by the motion sensor is also reported in a form of a data packet, that is, the smartwatch buffers, in the second buffer, the motion data in the motion data packet collected by the sensor. The motion data packet may include motion data of a preset numerical value. In other words, the motion sensor collects the motion data of the preset numerical value before reporting. For example, the motion sensor includes an acceleration sensor. Each time the acceleration sensor collects Q pieces of acceleration data, the acceleration sensor reports the Q pieces of acceleration data as an acceleration data packet. In other words, the acceleration data includes the Q pieces of acceleration data. For another example, the motion sensor includes a gyroscope. Each time the gyroscope collects T pieces of angular velocity data, the gyroscope reports the T pieces of angular velocity data as an angular velocity data packet. In other words, the acceleration data includes T pieces of acceleration data.

For example, a quantity of pieces of data included in the data packet may not change, or may change. For example, P is a first preset numerical value, and the first preset numerical value may be a positive integer greater than 0. The first preset numerical value may not change. When the smartwatch is in different modes, the first preset numerical value is the same. Certainly, to better reduce system overheads and power consumption of the smartwatch, the first preset numerical value may change. The first preset numerical value is related to a mode in which the smartwatch is located, and different modes have corresponding first preset numerical values. For example, when the smartwatch is in a motion mode, the first preset numerical value may be 10, that is, the PPG sensor collects every 10 pieces of PPG data before reporting. When the smartwatch is in a sleep mode, the first preset numerical value may be 20, that is, the PPG sensor collects every 20 pieces of PPG data before reporting. Similarly, Q is a second preset numerical value, and T is a third preset numerical value. The second preset numerical value may not change, or may change. The third preset numerical value may not change, or may change.

In some embodiments, if the PPG sensor reports the PPG data in a form of a data packet, a timestamp of the PPG data is actually a timestamp of the PPG data packet in which the PPG data is located. In other words, timestamps of all pieces of PPG data in one PPG data packet are the same, and the timestamps of all pieces of PPG data are the timestamp of the PPG data packet.

The PPG data in the PPG data packet may be arranged in a sequence of collection times from early to late, and PPG data with an earlier collection time has a higher ranking, and PPG data with a later collection time has a lower ranking. The timestamp of the PPG data packet may be a timestamp of one piece of PPG data in the PPG data packet. In an example, to ensure accuracy of data alignment, the piece of PPG data may be PPG data in a first preset sequence in the PPG data packet, and the first preset sequence may be a value of 1-P. For example, the first preset sequence is the first value, that is, the timestamp of each PPG data packet reported by the PPG sensor is the timestamp of the first piece of collected PPG data in the corresponding data packet. For another example, the first preset sequence is the last value, that is, the timestamp of each PPG data packet reported by the PPG sensor is the timestamp of the last piece of collected PPG data in the corresponding data packet.

In another example, because the PPG sensor collects data at a relatively high frequency and a shorter collection time interval, the foregoing piece of PPG data may be any piece of PPG data in the PPG data packet. For example, a timestamp of one PPG data packet is a timestamp of the first piece of collected PPG data in the PPG data packet. A timestamp of another PPG data packet is a timestamp of the third piece of collected PPG data in the PPG data packet.

Similarly, if the motion sensor reports the motion data in a form of a data packet, a timestamp of the motion data is actually a timestamp of the motion data packet in which the motion data is located. In other words, timestamps of all pieces of motion data in one motion data packet are the same, and the timestamps of all pieces of motion data are the timestamp of the motion data packet. A manner of determining the timestamp of the motion data packet is similar to the manner of determining the timestamp of the PPG data packet. For example, the motion sensor includes an acceleration sensor. Correspondingly, the motion data includes acceleration data, and the motion data packet includes an acceleration data packet. The timestamp of the acceleration data packet may be a timestamp of one piece of acceleration data in the acceleration data packet. In an example, to ensure accuracy of data alignment, the piece of acceleration data may be acceleration data in a second preset sequence in the acceleration data packet, and the second preset sequence may be a value of 1-Q. In another example, because the acceleration sensor collects data at a relatively high frequency and a shorter collection time interval, the piece of acceleration data may be any piece of acceleration data in the acceleration data packet.

It should be noted that a value of M may be fixed, and the value of M may also be related to a health function implemented by the smartwatch. In other words, the value of M may change, and the smartwatch may query, by using a related data table, a specific value of M corresponding to the implemented health function. For example, when the foregoing first operation is used to trigger the smartwatch to implement the heart rate monitoring function, the value of M may be 100. When the foregoing first operation is used to trigger the smartwatch to implement the sleep quality monitoring function, the value of M may be 150.

It may be determined from the foregoing description that the PPG sensor and each motion sensor in the smartwatch collect corresponding data at a specific frequency, that is, at a specific time interval. In other words, both the PPG sensor and each motion sensor have corresponding data collection frequencies. For ease of description, in this application, the frequency of the PPG sensor may be used as a first component frequency, and the frequency of each motion sensor may be separately used as a second component frequency of each motion sensor. The first component frequency may be the same as or different from the second component frequency. In addition, the second component frequencies of different motion sensors may also be the same or different, which is not limited in this application.

In one case, the component frequency of the sensor is fixed. When the smartwatch is in different modes, the first component frequency of the PPG sensor in the smartwatch is a same numerical value, and the second component frequency of the motion sensor in the smartwatch is also a same numerical value. For example, when the smartwatch is in a sleep mode, the first component frequency of the PPG sensor is 100 hz, and the second component frequency of the motion sensor is 100 hz. When the smartwatch is in a sleep mode, the first component frequency of the PPG sensor is still 100 hz, and the second component frequency of the motion sensor is still 100 hz.

In this case, the component frequency of the sensor may be preset, that is, the smartwatch pre-stores the first component frequency of the PPG sensor and the second component frequency of each motion sensor. The component frequency of the sensor may alternatively be obtained, after the smartwatch performs a specific operation (such as restart, algorithm switching, or mode switching), through prediction based on the first data packet and the second data packet that are reported by the sensor. Specifically, the smartwatch may obtain a component frequency through calculation based on the first data packet and the second data packet that are reported by the PPG sensor, and use the component frequency as the first component frequency of the PPG sensor. Similarly, for each motion sensor, the smartwatch may obtain a component frequency through calculation based on the first data packet and the second data packet that are reported by the motion sensor, and use the component frequency as the second component frequency of the motion sensor.

For example, the foregoing component frequency obtained through calculation based on the first data packet and the second data packet is specifically as follows: (Timestamp of the second data packet - Timestamp of the first data packet)/Quantity of pieces of data in the first data packet (or the second data packet). For example, one PPG data packet includes 10 pieces of PPG data. A collection time recorded by a timestamp of the first data packet collected by the smartwatch by using the PPG sensor after restart is a time point 1, and a collection time recorded by a timestamp of the second data packet is a time point 2. In this case, the smartwatch obtains the first component frequency of the PPG sensor based on (Time point 2 - Time point 1)/10.

In another case, the component frequency of the sensor is changeable, which is related to a current mode of the smartwatch. In different modes, component frequencies of the PPG sensor and the motion sensor are different. For example, when the smartwatch is in a sleep mode, the first component frequency of the PPG sensor is 100 hz, and the second component frequency of the motion sensor is 100 hz. When the smartwatch is in a sleep mode, the first component frequency of the PPG sensor is 70 hz, and the second component frequency of the motion sensor is 50 hz.

In this case, a process of determining the component frequency of the sensor is similar to the process of determining the foregoing component frequency. Specifically, the component frequency of the sensor may be preset, that is, the smartwatch pre-stores the first component frequencies of the PPG sensor in different modes and the second component frequency of each motion sensor. The component frequency of the sensor may alternatively be obtained, after the smartwatch performs a specific operation (such as mode switching), through prediction based on the first data packet and the second data packet that are reported by the sensor.

In this case, the first preset value corresponding to the motion sensor may be related to the component frequencies of the motion sensor and the PPG sensor. For example, the first preset value corresponding to the motion sensor depends on a difference between the first component frequency of the PPG sensor and the second component frequency of the motion sensor. A higher component frequency (such as the first component frequency and the second component frequency) of the sensor (such as the PPG sensor and the motion sensor) indicates a shorter time interval for data collection, that is, indicates a larger quantity of pieces of data collected by the sensor within a unit time. For example, the component frequency of the PPG sensor is 50 hertz (hertz, hz), which indicates that the PPG sensor collects 50 pieces of PPG data in 1 second. For another example, the component frequency of the PPG sensor is 100 hz, which indicates that the PPG sensor collects 100 pieces of PPG data in 1 second.

Therefore, when the difference between the first component frequency of the PPG sensor and the second component frequency of the motion sensor is larger, it indicates that the difference between the quantity of pieces of data collected by the PPG sensor within the unit time and the quantity of pieces of data collected by the motion sensor within the unit time is also larger, and correspondingly, the first preset value used to determine whether the motion data meets the preset condition may be larger. When the difference between the first component frequency of the PPG sensor and the second component frequency of the motion sensor is smaller, it indicates that the difference between the quantity of pieces of data collected by the PPG sensor within the unit time and the quantity of pieces of data collected by the motion sensor within the unit time is smaller, and correspondingly, the first preset value corresponding to the motion sensor may be smaller, so as to ensure that sufficient motion data can be fused, thereby improving accuracy of physiological parameter detection. For example, the component frequency of the PPG sensor is 50 hz, the second component frequency of the motion sensor is 25 hz, and the difference between the quantity of pieces of data collected by the PPG sensor within the unit time (for example, 1 second) and the quantity of pieces of data collected by the motion sensor within the unit time (for example, 1 second) is 25 pieces. However, when the component frequency of the PPG sensor is 100 hz, and the second component frequency of the motion sensor is 70 hz, the difference between the quantity of pieces of data collected by the PPG sensor within the unit time (for example, 1 second) and the quantity of pieces of data collected by the motion sensor within the unit time (for example, 1 second) is 30 pieces, which are more than 25 pieces. Therefore, the first preset value corresponding to the motion sensor when the first component frequency of the PPG sensor is 100 hz and the second component frequency of the motion sensor is 70 may be larger than the first preset value corresponding to the motion sensor when the first component frequency of the PPG sensor is 50 hz and the second component frequency of the motion sensor is 25 hz.

For example, for each motion sensor, after the smartwatch obtains, through calculation, the component frequency difference between the first component frequency of the PPG sensor and the second component frequency of the motion sensor, the smartwatch may search a preset data table for a first preset value corresponding to the component frequency difference, and use the first preset value as the first preset value corresponding to the motion sensor.

In some embodiments, to improve efficiency of multi-source data alignment, before performing multi-source data alignment, that is, before performing the steps of "storing the PPG data collected by the PPG sensor in a first buffer" and "storing the motion data collected by the motion sensor in a second buffer corresponding to the motion sensor" in S402, the smartwatch performs frequency offset check on the PPG data packet collected by the PPG sensor and the PPG data collected by each motion sensor, to obtain a frequency offset check result of the PPG sensor and a frequency offset check result of each motion sensor. The frequency offset check result indicates whether the frequency is abnormal.

If frequency offset check results of all sensors (namely, the PPG sensor and all motion sensors) indicate that a frequency is normal, the intelligent device may store data packets collected by the sensors in corresponding buffers (such as the first buffer and the second buffer), so as to avoid accumulation of a frequency offset error, thereby improving accuracy of multi-source data alignment and further improving accuracy of physiological parameter calculation.

When a frequency offset check result of a sensor indicates that a frequency is abnormal, it indicates that a packet loss phenomenon may occur, and a data packet collected by the sensor is abnormal. In this case, the smartwatch may not store the data collected by the sensor in a buffer corresponding to the sensor. For example, after collecting one PPG data packet by using the PPG sensor, the smartwatch performs frequency offset check on the PPG data packet to obtain a frequency offset check result of the PPG sensor. When the frequency offset check result of the PPG sensor indicates that the frequency of the PPG sensor is abnormal, it indicates that a PPG data packet loss may occur. In this case, the smartwatch may not store the data packet in the first buffer.

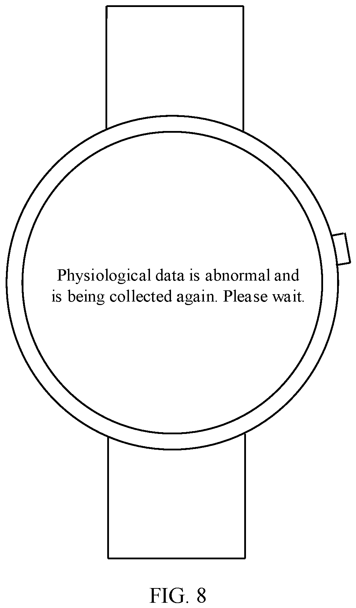

In addition, in the foregoing case in which the frequency offset check result of the sensor indicates that the frequency is abnormal, the smartwatch may further report an error. The smartwatch outputs second prompt information, where the second prompt information is used to indicate that physiological data is abnormal, so as to prompt the user to continue waiting for the smartwatch to calculate a physiological detection result (for example, second prompt information shown in FIG. 8).

The following describes the frequency offset check with reference to a specific example.

For the PPG sensor, the PPG data packet reported by the PPG sensor includes P pieces of PPG data. Each time the smartwatch collects one PPG data packet reported by the PPG sensor, the smartwatch may calculate a time difference between a timestamp of the PPG data packet and a timestamp of a previous PPG data packet of the PPG data packet (that is, timestamps of adjacent PPG data packets), and obtain an actual frequency of the PPG sensor based on the time difference/P. Then, the smartwatch calculates a difference between the first component frequency of the PPG sensor and the actual frequency of the PPG sensor. If the difference is less than a second preset value, it indicates that no PPG data packet loss phenomenon occurs between the adjacent PPG data packets. In this case, the smartwatch may determine that a frequency offset check result of the PPG sensor indicates that the frequency of the PPG sensor is normal. If the difference is greater than or equal to a third preset value, it indicates that a PPG data packet loss phenomenon occurs between the adjacent PPG data packets. In this case, the smartwatch may determine that a frequency offset check result of the PPG sensor indicates that the frequency of the PPG sensor is abnormal.

Similarly, for each motion sensor, each time the smartwatch collects one motion data packet collected by the motion sensor, the smartwatch may calculate a time difference between a timestamp of the motion data packet and a timestamp of a previous motion data packet of the motion data packet (that is, timestamps of adjacent motion data packets), and obtain an actual frequency of the motion sensor based on the time difference/a quantity of pieces of motion data in the motion data packet. Then, the smartwatch calculates a difference between the second component frequency of the motion sensor and the actual frequency of the motion sensor. If the difference is less than a third preset value, it indicates that no motion data packet loss phenomenon occurs between the adjacent motion data packets. In this case, the smartwatch may determine that a frequency offset check result of the motion sensor indicates that the frequency of the motion sensor is normal. If the difference is greater than or equal to a third preset value, it indicates that a motion data packet loss phenomenon occurs between the adjacent motion data packets. In this case, the smartwatch may determine that a frequency offset check result of the motion sensor indicates that the frequency of the motion sensor is abnormal.

In some embodiments, the smartwatch may store all data packets reported by the sensor in a buffer at the HAL layer (as shown in FIG. 3). For example, the HAL layer includes a buffer corresponding to the PPG sensor, and the buffer corresponding to the PPG sensor is used as a third buffer. The third buffer is configured to store all PPG data collected by the PPG sensor, that is, configured to store all PPG data packets reported by the PPG sensor. Then, the smartwatch may continuously read the PPG data packet from the third buffer. Each time the smartwatch reads one PPG data packet from the third buffer, the smartwatch performs frequency offset check on the PPG data packet, that is, performs frequency check on the PPG sensor. When a frequency offset check result of the PPG sensor indicates that a frequency is normal, it indicates that PPG data in the read PPG data packet is normal data. The smartwatch may buffer the read PPG data packet to the foregoing first buffer, so as to be used for multi-source data alignment. When the frequency offset check result of the PPG sensor indicates that the frequency is abnormal, it indicates that PPG data in the read PPG data packet is abnormal data. The smartwatch stops buffering the read PPG data packet to the first buffer. The smartwatch may continue to read a next PPG data packet from the third buffer.

Similarly, the HAL layer includes a buffer corresponding to each motion sensor, and the buffer corresponding to each motion sensor is used as a fourth buffer corresponding to the motion sensor. For the fourth buffer corresponding to each motion sensor, the fourth buffer corresponding to the motion sensor is configured to store all motion data collected by the motion sensor, that is, configured to store all motion data packets reported by the motion sensor. Then, the smartwatch may continuously read the motion data packet from the fourth buffer corresponding to the motion sensor. Each time the smartwatch reads one motion data packet from the fourth buffer corresponding to the motion sensor, the smartwatch performs frequency offset check on the motion data packet, that is, performs frequency check on the motion sensor. When the frequency offset check result of the motion sensor indicates that the frequency is normal, it indicates that the motion data in the read motion data packet is normal data. The smartwatch may buffer the read motion data packet to the second buffer corresponding to the motion sensor, so as to be used for multi-source data alignment. When the frequency offset check result of the motion sensor indicates that the frequency is abnormal, it indicates that the motion data in the read motion data packet is abnormal data. The smartwatch stops buffering the read motion data packet to the second buffer corresponding to the motion sensor. The smartwatch may continue to read a next motion data packet from the second buffer corresponding to the motion sensor.

It should be noted that when the frequency offset check result of the PPG sensor or the motion sensor indicates that the frequency is abnormal, the smartwatch may output the second prompt information.

S404. The smartwatch obtains and outputs a physiological detection result based on the M pieces of PPG data and a plurality of pieces of motion data that meet the preset condition and that are obtained from each second buffer.

For example, the foregoing physiological detection result may include a physiological parameter of the wearer of the smartwatch, for example, a specific parameter value such as a heart rate, blood oxygen, a cardiovascular, and a pulse of the wearer. The foregoing physiological detection result may include a health monitoring result of the wearer of the smartwatch, for example, sleep quality, a physical examination report, and a risk probability of a disease (for example, a probability that the wearer has an atrial fibrillation premature beat) of the wearer.

In this embodiment of this application, the smartwatch may first use the M pieces of PPG data and the plurality of pieces of motion data that meet the preset condition and that are obtained from each second buffer as aligned multi-source data. Then, the smartwatch fuses and denoises the aligned multi-source data, and can extract a real PPG waveform from the fused and denoised data, that is, a PPG waveform that does not include motion noise. Then, the intelligence may calculate a physiological parameter of the wearer of the smartwatch by using the real PPG waveform, so as to implement accurate detection of the physiological parameter. This can avoid a failure to accurately calculate the physiological parameter by using the PPG data due to motion noise, and can avoid a failure to accurately calculate the physiological parameter by using the multi-source data because the multi-source data is not aligned.

In some embodiments, if the smartwatch needs to display the physiological parameter of the wearer of the smartwatch, the foregoing physiological parameter obtained through calculation is the foregoing physiological detection result. In other words, the smartwatch may display the physiological parameter of the wearer after obtaining the physiological parameter through calculation.

In some other embodiments, if the smartwatch needs to display a health monitoring result of the wearer of the smartwatch, after obtaining the physiological parameter of the wearer through calculation, the smartwatch performs a health monitoring operation based on the physiological parameter of the wearer, for example, calculates a probability that the wearer has an atrial fibrillation premature beat based on a related algorithm model (such as an atrial fibrillation premature beat algorithm model) in the smartwatch. Herein, the probability that the wearer has an atrial fibrillation premature beat is the foregoing physiological detection result.

In some embodiments, the M pieces of PPG data and the plurality of pieces of motion data that meet the preset condition may alternatively be read by the smartwatch from the fifth buffer, so that the smartwatch can directly read the aligned multi-source data from the fifth buffer, and the smartwatch calculates a physiological detection result by using the aligned multi-source data, thereby improving efficiency of calculating the physiological detection result.

In this embodiment of this application, data layers and data verification of a plurality of dimensions may be used to detect abnormal data in a timely manner, and corresponding processing (for example, clearing motion data in the second buffer and stopping buffering the abnormal data to the first buffer/the second buffer) may be performed, so as to ensure accuracy of multi-source data for performing alignment processing, thereby ensuring accuracy of physiological parameter calculation.

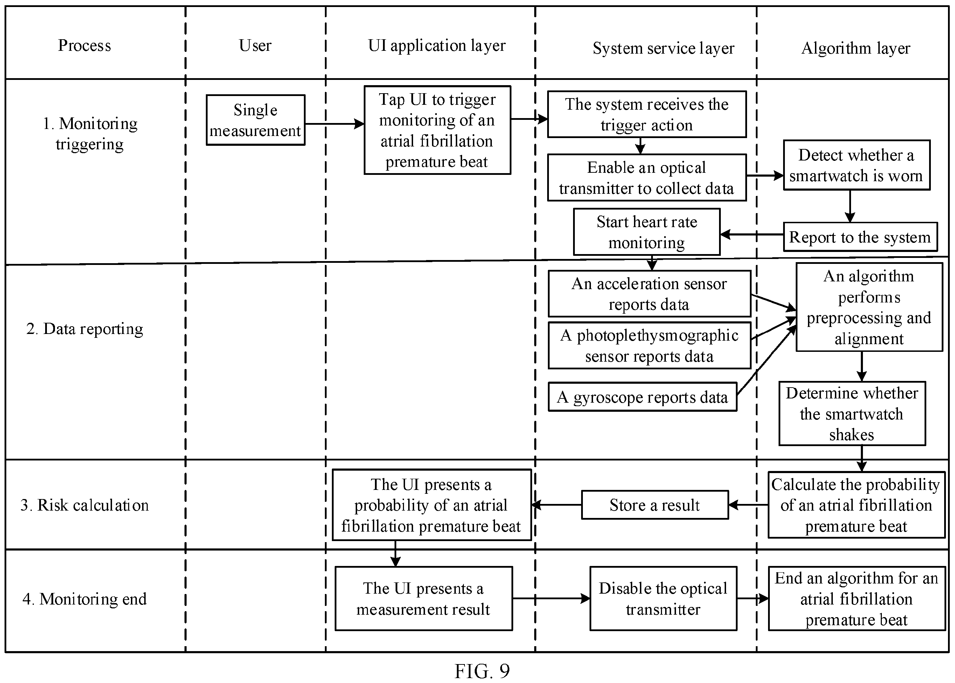

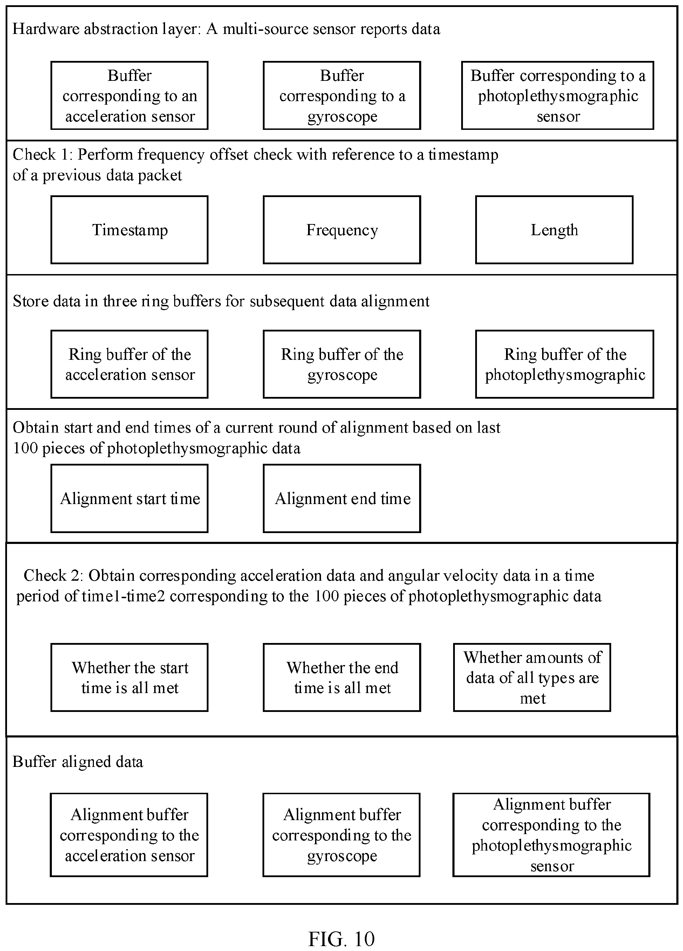

With reference to FIG. 9 and FIG. 10, the following specifically describes a possible process of calculating a physiological detection result by using multi-source data according to this application. The process sequentially includes a monitoring triggering process, a data reporting process, a risk calculation process, and a monitoring end process.

For example, as shown in FIG. 9, first, the smartwatch performs the monitoring triggering process. In the monitoring triggering process, a user wants the smartwatch to perform a single measurement, for example, to detect once a probability that a wearer of the smartwatch has an atrial fibrillation premature beat, and the user may tap an icon of an atrial fibrillation premature beat monitoring application in an interface displayed on the smartwatch. After receiving the tap operation, a system service layer of the smartwatch triggers an optical transmitter (that is, a PPG indicator) of the smartwatch to enable and start to collect wearing data, and sends the wearing data to an algorithm layer in the smartwatch. The algorithm layer detects, based on the wearing data, whether the smartwatch is worn, to obtain a wearing detection result, and reports the wearing detection result to the system service layer. In a case in which the wearing detection result indicates that the smartwatch is not worn, the system service layer may output prompt information used to prompt the user to wear the smartwatch.

In a case in which the wearing detection result indicates that the smartwatch is worn, the system service layer starts heart rate monitoring. Then, the smartwatch performs the data reporting process.

In the data reporting process, first, the system service layer may obtain an acceleration data packet reported by an acceleration sensor, a PPG data packet reported by a PPG sensor, and an angular velocity data packet reported by a gyroscope. For example, as shown in FIG. 10, the system service layer may read, from the buffer corresponding to the acceleration sensor at the HAL layer, the acceleration data packet reported by the acceleration sensor, and a buffer corresponding to the acceleration sensor at the HAL layer is configured to store all acceleration data reported by the acceleration sensor. Similarly, the system service layer may read, from the buffer corresponding to the PPG sensor at the HAL layer, PPG data packet reported by the PPG sensor. In addition, the system service layer may read, from a buffer corresponding to the gyroscope at the HAL layer, a gyroscope data packet reported by the gyroscope. Then, the system service layer may send the obtained acceleration data packet reported by the acceleration sensor, the obtained PPG data packet reported by the PPG sensor, and the obtained angular velocity data packet reported by the gyroscope (that is, multi-source data) to the algorithm layer. Then, the algorithm layer may perform preprocessing and alignment processing on the multi-source data. After obtaining the aligned multi-source data, the algorithm layer may determine, by using the aligned multi-source data, whether the smartwatch shakes (that is, moves) during collection of the aligned multi-source data, fuse and denoise the aligned multi-source data, and extract a real PPG waveform to avoid impact of motion noise. Then, the smartwatch performs the risk calculation process. The algorithm layer continues to use the real PPG waveform to calculate the probability that the wearer has an atrial fibrillation premature beat. Then, the algorithm layer may send the probability that the wearer has an atrial fibrillation premature beat to the system service layer. The system service layer stores the probability that the wearer has an atrial fibrillation premature beat, and sends the probability that the wearer has an atrial fibrillation premature beat to the UI application layer. The UI application layer displays, on a display screen of the smartwatch, the probability that the wearer has an atrial fibrillation premature beat.

For example, as shown in FIG. 10, the foregoing preprocessing indicates frequency offset check. After reading one acceleration data packet from the buffer corresponding to the acceleration sensor at the HAL layer, the system service layer may send the acceleration data packet to the algorithm layer. The algorithm layer may calculate an actual frequency of the acceleration sensor by using a timestamp of the acceleration data packet and a length of the acceleration data packet (that is, a quantity of pieces of acceleration data included in the acceleration data packet), and determine a frequency offset check result of the acceleration sensor based on the actual frequency and a component frequency of the acceleration sensor. If the frequency offset check result of the acceleration sensor indicates that a frequency of the acceleration sensor is normal, it indicates that the motion data packet is normal data. In this case, the algorithm layer may store the acceleration data packet in a ring buffer corresponding to the acceleration sensor at the algorithm layer. Similarly, after the system service layer reads one PPG data packet from a buffer corresponding to the PPG sensor at the HAL layer, the algorithm layer may determine a frequency offset check result of the PPG sensor by using the PPG data packet. If the frequency offset check result of the PPG sensor indicates that the frequency of the PPG sensor is normal, the algorithm layer may store the PPG data packet in a ring buffer corresponding to the PPG sensor at the algorithm layer. In addition, after the system service layer reads one gyroscope data packet from a buffer corresponding to the gyroscope at the HAL layer, the algorithm layer may determine a frequency offset check result of the gyroscope by using the gyroscope data packet. If the frequency offset check result of the gyroscope indicates that the frequency of the gyroscope is normal, the algorithm layer may store the gyroscope data packet in a ring buffer corresponding to the PPG sensor at the algorithm layer.

The data in the foregoing ring buffer may be used to perform alignment processing of the multi-source data.

As shown in FIG. 10, M is 100. A specific process of the alignment processing is as follows: After the ring buffer corresponding to the PPG sensor includes 100 pieces of PPG data, the algorithm layer determines a start time in all timestamps corresponding to the 100 pieces of PPG data, to obtain an alignment start time (that is, time1) and an end time in all timestamps corresponding to the 100 pieces of PPG data, to obtain an alignment end time (that is, time2). Then, the algorithm layer may obtain, from the ring buffer corresponding to the acceleration sensor, the acceleration data whose timestamp is in a time period of time1-time2, and obtain a start time and an end time that are in all the timestamps corresponding to the acceleration data in the time period of time1-time2, to obtain the start time and the end time of the acceleration sensor. Then, the algorithm layer determines whether the start time of the acceleration sensor meets a requirement, that is, whether a difference between the start time and the alignment start time is relatively small. In addition, the algorithm layer determines whether the end time of the acceleration sensor meets a requirement, that is, whether a difference between the end time and the alignment end time is relatively small. Similarly, the algorithm layer may obtain, from the ring buffer corresponding to the gyroscope, the angular velocity data whose timestamp is in a time period of time1-time2, and obtain a start time and an end time that are in all the timestamps corresponding to the angular velocity data in the time period of time1-time2, to obtain the start time and the end time of the gyroscope. Then, the algorithm layer determines whether the start time of the gyroscope meets a requirement, that is, whether a difference between the start time and the alignment start time is relatively small. In addition, the algorithm layer determines whether the end time of the gyroscope meets a requirement, that is, whether a difference between the end time and the alignment end time is relatively small.

If the start time and the end time of the acceleration sensor, and the start time and the end time of the gyroscope all meet a requirement, acceleration data in the time period of time1-time2, angular velocity data in the time period of time1-time2, and the foregoing 100 pieces of PPG data are aligned data. The algorithm layer may buffer the aligned multi-source data by using the buffer at the algorithm layer, that is, store the acceleration data in the time period of time1-time2 as aligned data by using an alignment buffer corresponding to the acceleration sensor, store the angular velocity data in the time period of time1-time2 as aligned data by using an alignment buffer corresponding to the gyroscope, and store the 100 pieces of PPG data as aligned data by using an alignment buffer corresponding to the PPG sensor, so that the algorithm layer reads the aligned multi-source data.

Finally, the smartwatch performs the monitoring end process. After the UI application layer displays, for a period of time, the probability that the wearer has an atrial fibrillation premature beat, the UI application layer may stop displaying the probability that the wearer has an atrial fibrillation premature beat, and display prompt information indicating that measurement ends. Then, the system service layer turns off the PPG indicator on the smartwatch.

It should be noted that the buffers (for example, alignment buffers) corresponding to the sensors at the algorithm layer and the buffers corresponding to the sensors at the HAL layer may be ring buffers, or may be non-ring buffers, for example, linked lists, which are not limited in this application. The sensors include a PPG sensor, a gyroscope, and an acceleration sensor.

In some embodiments, this application provides a computer storage medium, including computer instructions. When the computer instructions are run on a wearable device, the wearable device is enabled to perform the foregoing method.

In some embodiments, this application provides a computer program product. When the computer program product runs on a wearable device, the wearable device is enabled to perform the foregoing method.

It may be clearly learned by a person skilled in the art from the foregoing descriptions of the implementations that for convenience and brevity of description, only division into the foregoing functional modules is used as an example for description. In actual application, the foregoing functions may be allocated to different functional modules for implementation based on a requirement, that is, an internal structure of an apparatus is divided into different functional modules, to complete all or some of the functions described above.

In the several embodiments provided in this application, it should be understood that the disclosed apparatus and method may be implemented in other manners. For example, the described apparatus embodiment is merely an example. For example, the module or unit division is merely logical function division. In actual implementation, there may be another division manner. For example, a plurality of units or components may be combined or integrated into another apparatus, or some features may be ignored or not performed. In addition, the displayed or discussed mutual couplings or direct couplings or communication connections may be implemented through some interfaces. The indirect couplings or communication connections between the apparatuses or units may be implemented in an electrical form, a mechanical form, or another form.

The units described as separate parts may or may not be physically separate, and parts displayed as units may be one or more physical units, may be located in one place, or may be distributed in a plurality of different places. Some or all of the units may be selected based on actual requirements to achieve the objectives of the solutions in the embodiments.

In addition, the functional units in the embodiments of this application may be integrated into one processing unit, each of the units may exist alone physically, or two or more units may be integrated into one unit. The integrated unit may be implemented in a form of hardware, or may be implemented in a form of a software functional unit.

When the integrated unit is implemented in the form of a software functional unit and sold or used as an independent product, the integrated unit may be stored in a readable storage medium. Based on such an understanding, the technical solutions of the embodiments of this application essentially, the part contributing to the conventional technology, or all or some of the technical solutions may be implemented in a form of a software product. The software product is stored in a storage medium and includes several instructions for enabling a device (which may be a single-chip microcomputer, a chip, or the like) or a processor (processor) to perform all or some of the steps of the methods described in the embodiments of this application. The foregoing storage medium includes various media that can store program code, for example, a USB flash drive, a removable hard disk, a read-only memory (read only memory, ROM), a random access memory (random access memory, RAM), a magnetic disk, or an optical disc.

The foregoing content is merely specific implementations of this application, but is not intended to limit the protection scope of this application. Any variation or replacement made within the technical scope disclosed in this application shall fall within the protection scope of this application. Therefore, the protection scope of this application shall be subject to the protection scope of the claims.

## Claims

1. A wearable device-based physiological detection method, wherein the wearable device comprises a multi-source sensor, the multi-source sensor comprises a photoplethysmographic PPG sensor and one or more motion sensors, and the method comprises:
receiving, by the wearable device, a first operation, wherein the first operation is used to trigger the wearable device to detect a physiological parameter of a wearer of the wearable device;
in response to the first operation, collecting, by the wearable device, PPG data by using the PPG sensor, storing, in a first buffer, the PPG data collected by the PPG sensor, collecting motion data by using the motion sensor, and separately storing, in one or more second buffers, motion data collected by each motion sensor, wherein each piece of PPG data collected by the PPG sensor and each piece of motion data collected by the motion sensor separately correspond to one timestamp, which is used to record a collection time of corresponding data;
separately obtaining, by the wearable device from each second buffer based on timestamps corresponding to M pieces of PPG data, a plurality of pieces of motion data that meet a preset condition after collecting the M pieces of PPG data in the first buffer, wherein M is a preset value, the plurality of pieces of motion data that meet the preset condition in each second buffer are continuously collected by a corresponding motion sensor, times recorded by timestamps corresponding to the plurality of pieces of motion data are later than a time recorded by a first timestamp, a time recorded by a timestamp corresponding to at least one piece of motion data is earlier than a time recorded by a second timestamp, and a difference between a quantity of the plurality of pieces of motion data and M is less than a first preset value; the first timestamp is a timestamp with an earliest recording time in the timestamps corresponding to the M pieces of PPG data, and the second timestamp is a timestamp with a latest recording time in the timestamps corresponding to the M pieces of PPG data; and
obtaining and outputting, by the wearable device, a physiological detection result based on the M pieces of PPG data and the plurality of pieces of motion data that meet the preset condition and that are obtained from the one or more second buffers.

2. The method according to claim 1, wherein the first buffer and the second buffer are ring buffers.

3. The method according to claim 1 or 2, wherein the separately obtaining, by the wearable device from each second buffer based on timestamps corresponding to M pieces of PPG data, a plurality of pieces of motion data that meet a preset condition after collecting the M pieces of PPG data in the first buffer comprises:
reading, by the wearable device, motion data in a time interval corresponding to the first timestamp and the second timestamp from the second buffer after collecting the M pieces of PPG data in the first buffer;
if the wearable device reads the plurality of pieces of motion data that meet the preset condition, using the plurality of pieces of motion data that meet the preset condition and the M pieces of PPG data to perform physiological detection; or
if the wearable device reads N pieces of motion data, sending, by the wearable device, first prompt information, wherein the first prompt information is used to indicate that the wearable device is collecting physiological data, N is less than M, and a difference between M and N is greater than or equal to the first preset value.

4. The method according to claim 3, wherein the method further comprises:
if the wearable device reads the N pieces of motion data, clearing, by the wearable device, all motion data in the second buffer.

5. The method according to any one of claims 1-4, wherein a frequency of collecting PPG data by the PPG sensor is a first component frequency, and a frequency of collecting motion data by the one or more motion sensors is one or more second component frequencies; and
the first component frequency is the same as the one or more second component frequencies, or the first component frequency is different from the one or more second component frequencies; the first preset value depends on a difference between the first component frequency and the one or more second component frequencies; a smaller difference between the first component frequency and the one or more second component frequencies leads to a smaller first preset value; and second component frequencies of collecting motion data by different motion sensors are the same or different.

6. The method according to claim 5, wherein before the storing, in a first buffer, the PPG data collected by the PPG sensor, the method further comprises:
using, by the wearable device, every P pieces of PPG data collected by the PPG sensor as one first data packet, and storing the first data packet and a timestamp of the first data packet in a third buffer, wherein the timestamp of the first data packet is a timestamp corresponding to one piece of PPG data in the P pieces of PPG data, P is a first preset numerical value, and the timestamp of the first data packet is used as a timestamp corresponding to each piece of PPG data in the corresponding P pieces of PPG data; and
calculating, by the wearable device based on P and times recorded by timestamps of adjacent first data packets in the third buffer, a first actual frequency of collecting PPG data by the PPG sensor;
the storing, in a first buffer, the PPG data collected by the PPG sensor comprises:
if a difference between the first actual frequency and the first component frequency is less than a second preset value, buffering, by the wearable device, the first data packet in the third buffer to the first buffer; and
the collecting the M pieces of PPG data in the first buffer comprises: collecting K first data packets in the first buffer, wherein M=K*P.

7. The method according to claim 6, wherein the method further comprises:
if the difference between the first actual frequency and the first component frequency is greater than or equal to the second preset value, sending, by the wearable device, second prompt information, wherein the second prompt information is used to indicate that physiological data of the wearable device is abnormal.

8. The method according to any one of claims 5-7, wherein the one or more motion sensors comprise an acceleration sensor and a gyroscope; the one or more second buffers comprise an acceleration buffer configured to store first motion data collected by the acceleration sensor and a gyroscope buffer configured to store second motion data collected by the gyroscope.

9. The method according to claim 8, wherein before the separately storing, in one or more second buffers, motion data collected by each motion sensor, the method further comprises:
using, by the wearable device, every Q pieces of first motion data collected by the acceleration sensor as one second data packet, and storing the second data packet and a timestamp of the second data packet in a fourth buffer, wherein the timestamp of the second data packet is a timestamp corresponding to one piece of first motion data in the Q pieces of first motion data, Q is a second preset numerical value, and the timestamp of the second data packet is used as a timestamp corresponding to each piece of first motion data in the corresponding Q pieces of first motion data; and
calculating, by the wearable device based on Q and times recorded by timestamps of adjacent second data packets in the fourth buffer, a second actual frequency of collecting the first motion data by the acceleration sensor; and
storing the first motion data in the acceleration buffer comprises:
if a difference between the second actual frequency and the second component frequency corresponding to the acceleration sensor is less than a third preset value, buffering, by the wearable device, the second data packet in the fourth buffer to the acceleration buffer, wherein
the plurality of pieces of first motion data that meet the preset condition comprise W second data packets, N=W*Q, and N is a quantity of pieces of the first motion data in the plurality of pieces of first motion data that meet the preset condition.

10. The method according to any one of claims 1-9, wherein after the separately obtaining, by the wearable device from each second buffer based on timestamps corresponding to M pieces of PPG data, a plurality of pieces of motion data that meet a preset condition after collecting the M pieces of PPG data in the first buffer, the method further comprises:
storing, by the wearable device in a fifth buffer, the M pieces of PPG data and the plurality of pieces of motion data that meet the preset condition; and
the obtaining and outputting, by the wearable device, a physiological detection result based on the M pieces of PPG data and the plurality of pieces of motion data that meet the preset condition and that are obtained from the one or more second buffers comprises:
reading, by the wearable device from the fifth buffer, the M pieces of PPG data and the plurality of pieces of motion data that meet the preset condition, and obtaining and outputting the physiological detection result based on the M pieces of PPG data and the plurality of pieces of motion data that meet the preset condition.

11. A wearable device, wherein the wearable device comprises a display screen, a memory, a multi-source sensor, and one or more processors; the display screen, the multi-source sensor, and the memory are coupled to the processor; the display screen is configured to display an image generated by the processor, the multi-source sensor comprises a PPG sensor and one or more motion sensors, the memory is configured to store computer program code, and the computer program code comprises computer instructions; and when the processor executes the computer instructions, the wearable device is enabled to perform the method according to any one of claims 1 to 10.

12. A computer-readable storage medium, comprising computer instructions, wherein when the computer instructions are run on a wearable device, the wearable device is enabled to perform the method according to any one of claims 1 to 10.
